(19) Europäisches Patentamt
European Patent Office
Office européen des brevets

(11) Publication number: 0 117 575 B1

(12) EUROPEAN PATENT SPECIFICATION

(45) Date of publication of the patent specification: 06.08.86

(51) Int. Cl.[4]: C 07 C 67/297, C 07 C 69/14, C 07 C 67/00

(21) Application number: 84200139.8

(22) Date of filing: 02.02.84

# (54) Process for the preparation of esters.

(30) Priority: 17.02.83 NL 8300597

(43) Date of publication of application: 05.09.84 Bulletin 84/36

(45) Publication of the grant of the patent: 06.08.86 Bulletin 86/32

(84) Designated Contracting States: BE DE FR GB IT NL

(56) References cited:
EP - A - 0 034 062
EP - A - 0 058 442
WO - A - 82/02712
US - A - 3 579 566

(73) Proprietor: SHELL INTERNATIONALE RESEARCH MAATSCHAPPIJ B.V., Carel van Bylandtlaan 30, NL-2596 HR Den Haag (NL)

(72) Inventor: Drent, Eit, Badhuisweg 3, NL-1031 CM Amsterdam (NL)

(74) Representative: Aalbers, Onno et al, P.O. Box 302, NL-2501 CH The Hague (NL)

EP 0 117 575 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The invention relates to a process for the preparation of an ester by reacting a carboxylic acid anhydride and/or a 1,1-diester with hydrogen in the presence of carbon monoxide and a homogeneous ruthenium-containing catalyst. The invention relates in particular to the preparation of ethyl acetate from acetic acid anhydride and/or ethylidene diacetate.

In PCT patent application WO82/02712, a process is described for the preparation of ethylidene diacetate and/or ethyl acetate, in which process acetic acid anhydride is hydrogenated in the presence of a rhodium or ruthenium compound, methyl iodide and optionally lithium iodide. If it is desired to prepare ethyl acetate with this process, a ruthenium compound must be employed in combination with a limited quantity of methyl iodide, preferably in the absence of litium iodide. As appears from Example 2, in this manner ethyl acetate can be obtained at a high formation rate. For example, in experiment 3 mentioned in Table 2, at a temperature of 160°C and a pressure of approximately 140 bar, ethyl acetate is formed at a formation rate of 559 g per g of ruthenium per hour, albeit with moderate selectivity. The hydrogenation, howerer, is carried out with hydrogen not mixed with carbon monoxide. This also applies to the process described in Example 2 of US patent specification 3 957 827, with which ethyl acetate is prepared by hydrogenation of acetic acid anhydride with hydrogen in the presence of $RuCl_2 [(C_6H_5)_3P]_3$.

It would be advantageous if it were possible to employ, instead of pure hydrogen, mixtures of hydrogen and carbon monoxide as are obtained in a variety of industrial processes. In known processes in which such a mixture is used in the hydrogenation of acetic acid anhydride, howerer, the rate at which ethyl acetate is formed is far too low for application on a technical scale. In this connection, reference may be made, for exemple, to Example 4 of US patent specification 3 579 566, in which ethyl acetate is produced at a rate of only 25 g per g of ruthenium per hour, and to European patent application 34062. In the latter patent application, a process is described for the preparation of ethylidene diacetate, in which process acetic acid anhydride is hydrogenated with hydrogen or a mixture of carbon monoxide and hydrogen in the presence of a catalyst containing as its main constituent a compound of a metal of Group VIII of the Periodic System, and as its second constituent a chloride, bromide or iodide chosen from the large group of alkyl or alkanoyl halogenides and the inorganic halogenides including the hydrogen halogenides, or ammonia or an organic nitrogen compound. In the examples where the reaction is carried out with a mixture of carbon monoxide and hydrogen, howerer, the second constituent used is invariably methyl iodide or an organic nitrogen compound. In most examples, ethylidene diacetate is formed as main product in addition to at most a minor quantity of ethyl acetate. Only in Example 22, where the hydrogenation is carried out in the presence of ruthenium chloride and 2,6-lutidine, is ethyl acetate formed in major quantities. The formation rate of the ethyl acetate, howerer, is very low, and is only 9.9 g per g of ruthenium per hour.

From UK patent specification 2 034 307 a process is known for the preparation of 1,1-diesters such as ethylidene diacetate, in which process a carboxylic acid anhydride is contacted with hydrogen in the presence of an insoluble (heterogeneous) hydrogenation catalyst and a strong protonic acid. With the exception of one example, in which Raney nickel is used as catalyst, palladium on carbon is invariably used in the examples. In the examples the hydrogenation is carried out in the absence of carbon monoxide. Only Example 13 reports the formation of a very minor quantity of ethyl acetate (selectivity 2%). In this example, the hydrogenation is carried out in the presence of acetyl fluoride as source of hydrogen fluoride. In European patent application 58442, a process is described for the preparation of diesters such as, for example, ethylidene diacetate, by reacting a carboxylic acid anhydride with hydrogen in the presence of carbon monoxide, a homogeneous Group VIII metal catalyst, a chloride, bromide or iodide and an organic compound of oxygen, nitrogen, phosphorus, arsenic or antimony with a lone pair of electrons. Formation of monoesters is not reported in European patent application 58442. Importantly, howerer, in Examples 14 and 15 it is demonstrated that the process of UK patent specification 2 034 037 cannot be carried out in the presence of carbon monoxide.

In the experimental section, it will be demonstrated that even if the hydrogenation of acetic acid anhydride is carried out with catalysts with which according to PCT application WO82/02712, in the absence of carbon monoxide, hitherto, the best results have been obtained, in particular ruthenium catalysts, addition of carbon monoxide causes the formation rate of ethyl acetate to fall very disadvantageously, and that this negative effect is further reinforced by the presence of methyl iodide, the promoter employed in that application.

It has now been found that if the hydrogenation of acetic acid anhydride with a mixture of carbon monoxide and hydrogen and a ruthenium catalyst is carried out in the presence of hydrogen chloride or hydrogen bromide, ethyl acetate is formed at a high rate suitable for practical application, and at a high selectivity. This is surprising because the addition of acids would not be expected to have any beneficial effect in the light of European patent application 34062, where the basic substance 2,6-lutidene is used as promoter for the formation of ethyl acetate, and US patent specification 3 579 566, where the likewise basic triphenyl phosphine is used. The strong promoter action of hydrogen chloride and hydrogen bromide was found to be specific to these compounds. After addition of hydrogen fluoride or benzene phosphonic acid, the formation rate of ethyl acetate was found to be no higher than in the absence of a promoter, while in the presence of hydrogen iodide or p-toluene sulphonic acid, ethylidene diacetate was formed virtually exclusively. Addition of trifluoro-acetic acid or fluoroboric acid, yielded only a relatively small increase in the formation rate of ethyl acetate, which is of little practical significance.

The invention therefore relates to a process for the preparation of an ester by reacting a carboxylic acid anhydride and/or 1,1-diester with hydrogen in the presence of carbon monoxide and a homogeneous ruthenium-containing catalyst, characterized in that the reaction is carried out in the presence of hydrogen chloride or hydrogen bromide. The hydrogen chloride or hydrogen bromide may be formed in situ by the reaction of a chlorine or bromine compound with carbon monoxide and/or hydrogen. Examples of such compounds are acetyl chloride and acetyl bromide.

The invention provides a new, synthesis gas-based process for the preparation of esters such as, for example, ethyl acetate, which is used on a large scale as a solvent for many materials, such as cellulose esters. The base materials which are used in the process according to the invention can likewise be produced with the aid of synthesis gas, which is important in connection with the present interest in processes for the preparation of chemicals from non-petroleum based materials. In relation to the process according to PCT application WO82/02712, the process according to the invention has the advantage that it is not necessary to use carbon monoxide-free hydrogen. The use of pure hydrogen is not only less attractive from the economic point of view, but as a result of the instability of the catalyst it may cause a deposit to form during the hydrogenation, which is very inconvenient especially during continuous operation of the process. Furthermore, the process according to the invention yields a gas stream with a higher carbon monoxide content than the starting synthesis gas, because the hydrogen present therein is consumed in the hydrogenation. This gas stream with an elevated CO content can be used, for example, in the preparation of the starting materials (carboxylic acid anhydride or 1,1-diester) by means of carbonylation reactions. The process according to the invention is therefore particularly suitable for integration with other chemical processes where carbon monoxide is used.

The quantity of hydrogen chloride or hydrogen bromide is preferably between 1 and 150, in particular between 1 and 50 and most preferably between 1 and 20 mol per gram atom of ruthenium .

The carboxylic acid anhydride and the 1,1-diester which are used as starting material in the process according to the invention, have the following formulas I and II, respectively

| I | II |
|---|---|
| R-CO-O-CO-R | R-CH< (O-CO-R)(O-CO-R) |

wherein the groups R are identical or non-identical, optionally substituted, hydrocarbon groups. The hydrocarbon groups may be alkyl, cycloalkyl, alkenyl, alkynyl, aryl, aralkyl or alkaryl groups containing preferably not more than 20 carton atoms. They may optionally be substituted with one or more substituents such as, for example, halogen atoms or alkoxy groups. If the hydrocarbon groups contain one or more olefinic double bonds, hydrogenation of these double bonds may take place under the conditions employed in the process according to the invention. The hydrocarbon groups are preferably unsubstituted alkyl groups with 1-6 carbon atoms. Acetic acid anhydride and ethylidene diacetate are very suitable starting materials.

When a carboxylic acid anhydride is hydrogenated, in the first instance one molecule of 1,1-diester and one molecule of carboxylic acid can be formed from two molecules of carboxylic acid anhydride. The 1,1-diester can subsequently be hydrogenated to form one molecule of monoester and a second molecule of carboxylic acid. The monoester, howerer, can also be formed directly from the carboxylic acid anhydride. For example, acetic acid anhydride can be converted into ethyl acetate directly or via ethylene diacetate. Howerer, it is a characteristic of the process according to the invention that any 1,1-diester formed is converted at a high rate into monoester.

The ratio between hydrogen and carbon monoxide can vary within wide limits. The molar ratio between hydrogen and carbon monoxide is preferably between 1:10 and 20:1, preferably between 1:2 and 15:1. The total pressure measured at room temperature is preferably between 10 and 150, in particular between 20 and 100 bar. Higher pressures, up to for example 1000 bar, may be used, but as a rule they are not particularly attractive for economic reasons. Other gases such as carbon dioxide, methane, nitrogen or noble gases may also be present in the carbon monoxide and hydrogen mixture.

The homogeneous ruthenium-containing catalyst may be, for example, a salt of a mineral acid, for example a halide, nitrate or sulphate, or a salt of an organic acid, for example of an alkane carboxylic acid with 1-20 carbon atoms, such as acetic acid or propionic acid. Complex compounds such as, for example carbonyl compounds may also be used. Examples of suitable ruthenium-containing catalysts are ruthenium nitrate, ruthenium acetate, ruthenium chloride, ruthenium bromide and $Ru_3(CO)_{12}$. The quantity of ruthenium used in the process according to the invention is preferably between $3 \cdot 10^{-6}$ and $10^{-1}$, in particular between $10^{-5}$ and $10^{-2}$, and most preferably between $3 \cdot 10^{-5}$ and $10^{-2}$ gram atoms of ruthenium per mol of carboxylic acid anhydride or 1,1-diester.

The reaction is preferably carried out in the absence of organic compounds of nitrogen, phosphorus, arsenic or antimony with a lone pair of electrons because they promote the formation of diester. Moreover, from PCT application WO82/02712 it is known that compounds of this type, such as the phosphines, are often unstable under the reaction conditions used in the hydrogenation of the carboxylic acid anhydride, and give rise to the formation of soot and tar which contaminate the catalyst and hamper its recovery.

It was surprisingly found, howerer, that in the process according to the invention certain compounds of pentavalent phosphorus, arsenic or antimony are suitable promoters for the formation of esters. Moreover, these compounds are stable under the reaction conditions used, so that no inconvenient decomposition products are formed.

The reaction is therefore preferably carried out in the presence of a compound having the formula

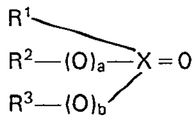

III

wherein X is phosphorus, arsenic or antimony and $R^1$ is hydrogen or an optionally substituted hydrocarbon group, and either a and b are independently 0 or 1, and $R^2$ and $R^3$ each represent an optionally substituted hydrocarbon group, or a and b are both 0 and $R^2$ and $R^3$ form together with X a heterocyclic group or in the presence of a complex of a compound having formula III with a hydrocarbon chloride or bromide, an acyl chloride or bromide, or hydrogen chloride or hydrogen bromide, which complexes can also be formed in situ.

The hydrocarbon groups $R^1$, $R^2$ and $R^3$ may be alkyl, cycloalkyl, aryl, aralkyl or alkaryl groups containing preferably not more than 30, in particular not more than 20, carbon atoms and are optionally substituted with one or more substituents, such as for example halogen atoms or a group $R^4R^5X=O$, where X has the significance indicated above and $R^4$ and $R^5$ each represent an optionally substituted hydrocarbon group. If $R^2$ and $R^3$ form a heterocyclic group with X, that group preferably contains not more than 20 carbon atoms. Specific examples are the phospholane, phosphorinane and phosphohepane group, where the groups $R^2$ and $R^3$ together represent an alkylene group with 4, 5 or 6 carbon atoms, respectively, and the 9-phosphabicyclo-[4.2.1]nonane group and the 9-phosphabicyclo-[3.3.1]nonane group. These heterocyclic groups may, for example, be substituted with hydrocarbon groups.

Compounds having the formula III, wherein a and b are 0, X is phosphorus and $R^1$, $R^2$ and $R^3$ are alkyl groups with 1-5 carbon atoms and/or cycloalkyl, aryl, aralkyl or alkaryl groups with 5-12 carbon atoms, are preferred. Special preference is given to compounds having the general formula III, wherein $R^1$, $R^2$ and $R^3$ represent alkyl groups with 1-12 carbon atoms and/or phenyl groups optionally substituted with one or more methyl or ethyl groups.

Specific examples of compounds having formula III are the oxides of secondary and tertiary phosphines, arsines and stibines such as trimethyl phosphine oxide, diethyl phosphine oxide, triethyl phosphine oxide, tri-n-butyl phosphine oxide, trioctyl phosphine oxide, diphenyl phosphine oxide, tri-p-tolyl phosphine oxide, tricyclohexyl phosphine oxide, diphenyl ethyl phosphine oxide, tri(1-naphthyl)-phosphine oxide, triethyl arsine oxide and triphenyl stibine oxide. Triphenyl phosphine oxide is a preferentially employed promoter. Specific examples of compounds with a heterocyclic phosphorus-containing group are 1-phenyl phospholane oxide, 1-phenyl phosphorinane oxide, 9-phenyl-9--phosphabicyclo[4.2.1]nonane oxide, 9-phenyl-9--phosphabicyclo[3.3.1]nonane oxide, 9-eicosyl-9--phosphabicyclo[4.2.1]nonane oxide and 9-eicosyl--9-phosphabicyclo[3.3.1]nonane oxide.

Examples of compounds having the general formula III, wherein a and/or b are 1, are the alkyl, cycloalkyl, aryl, aralkyl and alkaryl esters of phosphonic acids and phosphinic acids, and the analoga of these compounds where the phosphorus atom is replaced by an arsenic or antimony atom. Specific examples of such compounds are dimethyl methyl phosphonate, diethyl methyl phosphonate, diphenyl methyl phosphonate, methyl diethyl phosphinate and phenyl dimethyl phosphinate.

Specific examples of compounds having the formula III, wherein one or more of the groups $R^1$, $R^2$ and $R^3$ are substituted with a group $R^4R^5X=O$, are the compounds:

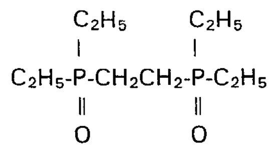

and

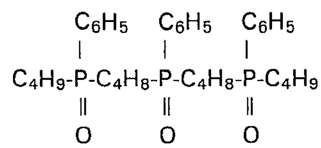

If, in the compound having formula III, X represents phosphorus and a and b are 0, this compound may be formed in situ using the corresponding phosphine in the place of the relevant compound having the general formula III, and performing the reaction in the presence of molecular oxygen or hydrogen peroxide.

The quantity of the compound having formula III or of its complex may vary between wide limits, for example between 0.1 and 300 mol per gram atom or ruthenium. Preferably 1-100, in particular 2-50 mol per gram atom of ruthenium is used. The process according to the invention is preferably carried out at a temperature between 110 and 225°C, in particular between 125 and 220°C. The reaction is preferably carried out under substantially anhydrous conditions in order to prevent the occurrence of hydrolysis of the starting material or of the resultant ester. The presence of minor quantities of water as is common in the commercially available chemicals employed in the process, for example in the form of water of crystallization, is nevertheless allowable. The reaction mixture preferably contains not more than 2, in particular not more than 0.2% by weight of water.

The process may be carried ou continuously, semi-continuously or batchwise. Generally is is not necessary to use a solvent because the starting material and/or the resultant ester and/or the carboxylic acid formed as a by-product act sufficiently as solvent. If desired, additional quantities of these compounds or an additional solvent may be added to the reaction mixture. The reaction mixture can be worked up with the aid of known techniques, such as, for example, fractional distillation.

*Exemple*

50 ml of acetic acid anhydride, 1 mmol $RuCl_3 \cdot 3H_2O$ and the quantities as stated in Table A of the

compounds X listed in colum 1 and triphenyl phosphine oxide were placed in a magnetically stirred autoclave of Hastelloy C (Hastelloy is a trade name) of 300 ml capacity. The autoclave was filled with hydrogen or a mixture of hydrogen and carbon monoxide at the pressure stated in Table A, and was subsequently heated to 160°C. After the reaction time mentioned in Table A the reaction mixture was cooled down and by means of gas-liquid chromatography the quantities of ethyl acetate, ethylidene diacetate and acetic acid were determined. The rate at which ethyl acetate was formed is shown in the last column of Table A. Experiments 1-3 and 8-14 are comparative experiments.

Comparison of experiments 1 and 2 shows that the presence of carbon monoxide has an extremely adverse effect on the formation rate of ethyl acetate, and comparison of experiments 2 and 3 shows that methyl iodide in the presence of carbon monoxide causes the formation rate of ethyl acetate to fall. From experiments 8, 9 and 10 it appears that hydrogen iodide and p-toluene sulphonic acid severely hamper the formation of ethyl acetate. Experiments 12 and 14 in comparison with experiment 3 show that hydrogen fluoride and benzenephosphonic acid scarcely increase the formation rate of ethyl acetate even in the presence of triphenyl phosphine oxide, and experiments 11 and 13 in comparison with experiment 3 demonstrate that the boosting effect of fluoroboric acid and trifluoroacetic acid on the formation rate of ethyl acetate is only relatively small. From experiments 5, 6 and 7 it appears that the addition of HCl or HBr in the presence of carbon monoxide allows ethyl acetate to be formed at a rate which equals or at least approaches the formation rate obtainable by hydrogenation with pure hydrogen, and moreover that the reaction proceeds with a high selectivity. Finally, the formation of a catalyst deposit in experiment 1 demonstrates that the ruthenium catalyst is less than stable in the absence of carbon monoxide.

TABLE A

| Experiment No. | Compound X (mmol) | $P_{CO}$ (bar) | $P_{H_2}$ (bar) | Reaction time (hours) | Triphenyl phosphine oxide (mmol) | Ethyl acetate (mol) | Ethylidene diacetate (mol) | Acetic acid (mol) | Formation rate of ethyl acetate in g/g Ru/hour |
|---|---|---|---|---|---|---|---|---|---|
| 1 | $CH_3J$ (2) | — | 60 | 0.5 | — | 0.18 | trace | 0.49 | $316^{+}$ |
| 2 | $CH_3J$ (2) | 15 | 50 | 1 | — | 0.035 | 0.11 | 0.20 | 30 |
| 3 | — — | 15 | 50 | 2 | — | 0.13 | 0.07 | 0.42 | 57 |
| 4 | HCl* (3) | 15 | 50 | 1 | — | 0.177 | 0.027 | 0.46 | 156 |
| 5 | HCl* (3) | 15 | 50 | 1 | 4 | 0.193 | 0.022 | 0.49 | 170 |
| 6 | HCl* (3) | 15 | 50 | 0.5 | 8 | 0.126 | 0.082 | 0.37 | 221 |
| 7 | HBr** (3) | 15 | 50 | 0.25 | 4 | 0.125 | 0.043 | 0.31 | 440 |
| 8 | HJ*** (3) | 15 | 50 | 0.5 | — | 0.01 | 0.048 | 0.20 | 18 |
| 9 | pTS° (3) | 15 | 50 | 1 | 4 | 0.03 | 0.083 | 0.25 | 26 |
| 10 | pTS° (6) | 15 | 50 | 1 | 4 | 0.03 | 0.04 | 0.46 | 26 |
| 11 | $HBF_4$°° (3) | 15 | 50 | 2 | 4 | 0.17 | 0.017 | 0.48 | 75 |
| 12 | HF°°° (3) | 15 | 50 | 2 | 4 | 0.14 | 0.09 | 0.45 | 62 |
| 13 | $CF_3COOH$ (3) | 15 | 50 | 1 | 4 | 0.088 | 0.072 | 0.29 | 77 |
| 14 | benzene phosphonic acid (3) | 15 | 50 | 2 | 4 | 0.14 | 0.084 | 0.47 | 62 |

* 37% by wt in $H_2O$
** 57% by wt in $H_2O$
*** 55% by wt in $H_2O$
° pTS = p-toluene sulphonic acid
°° 60% by wt in $H_2O$
°°° 40% by wt in $H_2O$
$^{+}$ formation of catalyst deposit

**Claims**

1. Process for the preparation of an ester by reacting a carboxylic acid anhydride and/or 1,1-diester with hydrogen in the presence of carbon monoxide and a homogeneous ruthenium-containing catalyst, characterized in that the reaction is carried out in the presence of hydrogen chloride or hydrogen bromide.

2. Process as claimed in claim 1, characterized in that the quantity of hydrogen chloride or bromide is between 1 and 50 mol per gram atom of ruthenium.

3. Process as claimed in claim 1, characterized in that the carboxylic acid anhydride and the 1,1-diester have the respective formulas

R-CO-O-CO-R (I)

$$R\text{-}CH\begin{cases}O\text{-}CO\text{-}R\\O\text{-}CO\text{-}R\end{cases}$$ (II)

wherein the groups R are identical or non-identical, optionally substituted, hydrocarbon groups.

4. Process as claimed in claim 3, characterized in that the hydrocarbon groups are unsubstituted alkyl groups with 1-6 carbon atoms.

5. Process as claimed in claims 1-4, characterized in that the molar ratio between hydrogen and carbon monoxide is beetween 1:2 and 15:1.

6. Process as claimed in claims 1-5, characterized in that the reaction is carried out at a total pressure, measured at room temperature, of beetween 10 and 150 bar.

7. Process as claimed in claims 1-6, characterized in that the reaction is carried out in the presence of a compound having the formula

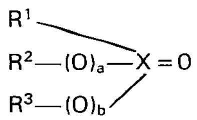

III

wherein X is phosphorus, arsenic or antimony and $R^1$ is hydrogen or an optionally substituted hydrocarbon group and either a and b are independently 0 or 1 and $R^2$ and $R^3$ each represent an optionally substituted hydrocarbon group, or a and b are both 0 and $R^2$ and $R^3$ form together with X a heterocyclic group, or in the presence of a complex of a compound having the formula III with a hydrocarbon chloride or bromide, an acyl chloride or bromide or a hydrogen chloride or bromide.

8. Process as claimed in claim 7, characterized in that the hydrocarbon groups $R^1$, $R^2$ and $R^3$ are alkyl, cycloalkyl, aryl, aralkyl or alkaryl groups with not more than 30 carbon atoms.

9. Process as claimed in claim 7, characterized in that $R^2$ and $R^3$ together with X form a heterocyclic group with not more than 20 carbon atoms.

10. Process as claimed in claim 7 or 8, characterized in that a and b are 0, X is phosphorus and $R^1$, $R^2$ and $R^3$ represent alkyl groups with 1-12 carbon atoms and/or cycloalkyl, aryl, aralkyl or alkaryl groups with 5-12 carbon atoms.

11. Process as claimed in claim 10, characterized in that $R^1$, $R^2$ and $R^3$ represent alkyl groups with 1-12 carbon atoms and/or phenyl groups optionally substituted with one or more methyl or ethyl groups.

12. Process as claimed in claims 1-11, characterized in that the reaction is carried out a temperature between 110 and 225°C, in particular between 125 and 200°C.

**Patentansprüche**

1. Verfahren zur Herstellung eines Esters durch Umsetzung eines Carbonsäureanhydrids und/oder eines 1,1-Diesters mit Wasserstoff in Gegenwart von Kohlenmonoxid und einem homogenen Ruthenium-haltigen Katalysator, dadurch gekennzeichnet, dass die Reaktion in Gegenwart von Chlorwasserstoff oder Bromwasserstoff durchgeführt wird.

2. Verfahren nach Anspruch 1, dadurch gekennzeichnet, dass die Menge an Chlorwasserstoff oder Bromwasserstoff zwischen 1 und 50 mol pro g Atom Ruthenium beträgt.

3. Verfahren nach Anspruch 1, dadurch gekennzeichnet, dass das Carbonsäureanhydrid und der 1,1-Diester den folgenden Formeln entsprechen,

R-CO-O-CO-R

I

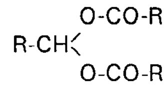

II

wobei die Gruppen R gleiche oder ungleiche, gegebenenfalls substituierte Kohlenwasserstoff-Gruppen sind.

4. Verfahren nach Anspruch 3, dadurch gekennzeichnet, dass die Kohlenwasserstoffgruppen unsubstituierte Alkyl-Gruppen mit 1 bis 6 Kohlenstoffatomen sind.

5. Verfahren nach Anspruch 1 bis 4, dadurch gekennzeichnet, dass das Molverhältnis zwischen Wasserstoff und Kohlenmonoxid zwischen 1:2 und 15:1 liegt.

6. Verfahren nach Anspruch 1 bis 5, dadurch gekennzeichnet, dass die Reaktion bei einem Gesamtdruck, gemessen bei Raumtemperatur, zwischen 10 und 150 bar durchgeführt wird.

7. Verfahren nach Anspruch 1 bis 6, dadurch gekennzeichnet, dass die Reaktion durchgeführt wird in Gegenwart einer Verbindung der Formel

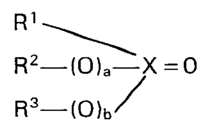

III

in der X Phosphor, Arsen oder Antimon ist und $R^1$ Wasserstoff oder eine gegebenenfalls substituierte Kohlenwasserstoff-Gruppe und a und b unabhängig von einander 0 oder 1 bedeuten und $R^2$ und $R^3$ jeweils eine gegebenenfalls substituierte Kohlenwasserstoff-Gruppe bedeuten oder a und b beide 0 sind und $R^2$ und $R^3$ zusammen mit X eine heterocyclische Gruppe bilden, oder in Gegenwart eines Komplexes einer Verbindung der Formel III mit einem Kohlenwasserstoffchlorid oder -bromid, einem Acylchlorid oder -bromid oder Chlorwasserstoff oder Bromwasserstoff.

8. Verfahren nach Anspruch 7, dadurch gekennzeichnet, dass die Kohlenwasserstoff-Gruppen $R^1$, $R^2$ und $R^3$ Alkyl-, Cycloalkyl-, Aryl-, Aralkyl- oder Alkaryl-Gruppen mit nicht mehr als 30 Kohlenstoffatomen sind.

9. Verfahren nach Anspruch 7, dadurch gekennzeichnet, dass $R^2$ und $R^3$ zusammen mit X eine heterocyclische Gruppe mit nicht mehr als 20 Kohlenstoffatomen bilden.

10. Verfahren nach Anspruch 7 oder 8, dadurch gekennzeichnet, dass a und b 0 sind, X Phosphor und $R^1$, $R^2$ und $R^3$ (jeweils) Alkylgruppen mit 1 bis 12 Kohlenstoffatomen und/oder Cycloalkyl-, Aryl,-Aralkyl- oder Alkylaryl-Gruppen mit 5 bis 12 Kohlenstoffatomen bedeuten.

11. Verfahren nach Anspruch 10, dadurch gekennzeichnet, dass $R^1$, $R^2$ und $R^3$ (jeweils) Alkyl-Gruppen mit 1 bis 12 Kohlenstoffatomen und/oder Phenyl-Gruppen, die gegebenenfalls durch eine oder

mehrere Methyl- oder Ethyl-Gruppen substituiert sind, bedeuten.

12. Verfahren nach Anspruch 1 bis 11, dadurch gekennzeichnet, dass die Reaktion bei einer Temperatur zwischen 110 und 225°C, insbesondere zwischen 125 und 200°C durchgeführt wird.

## Revendications

1. Procédé pour la préparation d'un ester par réaction d'un anhydride d'acide carboxylique et/ou d'un 1,1-diester avec l'hydrogène en présence d'oxyde de carbone et d'un catalyseur homogène contenant du ruthénium, caractérisé en ce que la réaction est conduite en présence d'acide chlorhydrique ou d'acide bromhydrique.

2. Procédé selon le revendication 1, caractérisé en ce que la quantité d'acide chlorhydrique ou d'acide bromhydrique est comprise entre 1 et 50 moles par atome-gramme de ruthénium.

3. Procédé selon la revendication 1, caractérisé en ce que l'anhydride d'acide carboxylique et le 1,1-diester ont les formules respectives

O-CO-R
R-CO-O-CO-R R-CH<
O-CO-R

I II

où les groupes R sont des groupes d'hydrocarbures éventuellement substitués identiques ou différents.

4. Procédé selon la revendication 3, caractérisé en ce que les groupes d'hydrocarbures sont des groupes alcoyle non-substitués ayant 1-6 atomes de carbone.

5. Procédé selon les revendications 1-4, caractérisé en ce que le rapport molaire entre l'hydrogène et l'oxyde de carbone est compris entre 1:2 et 15:1.

6. Procédé selon les revendications 1-5, caractérisé en ce que la réaction est conduite à une pression totale, mesurée à la température ambiante, comprise entre 10 et 150 bars.

7. Procédé selon les revendications 1-6, caractérisé en ce que la réaction est conduite en présence d'un composé ayant la formule

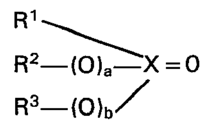

III

où X est du phosphore, de l'arsenic ou de l'antimoine et $R^1$ est de l'hydrogène ou un groupe d'hydrocarbure éventuellement substitué et soit a et b sont indépendamment 0 ou 1 et $R^2$ et $R^3$ représentent chacun un groupe d'hydrocarbure éventuellement substitué, soit a et b sont tous deux 0 et $R^2$ et $R^3$ forment ensemble avec X un groupe hétérocyclique, ou en présence d'un complexe d'un composé ayant la formule III avec un chlorure ou bromure d'hydrocarbure, un chlorure ou bromure d'acyle ou l'acide chlorhydrique ou bromhydrique.

8. Procédé selon la revendication 7, caractérisé en ce que les groupes d'hydrocarbures $R^1$, $R^2$ et $R^3$ sont des groupes alcoyle, cycloalcoyle, aryle, aralcoyle ou alcaryle n'ayant pas plus de 30 atomes de carbone.

9. Procédé selon la revendication 7, caractérisé en ce que $R^2$ et $R^3$ forment ensemble avec X un groupe hétérocyclique n'ayant pas plus de 20 atomes de carbone.

10. Procédé selon la revendication 7 ou 8, caractérisé en ce que a et b sont 0, X est du phosphore et $R^1$, $R^2$ et $R^3$ représentent des groupes alcoyle de 1-12 atomes de carbone et/ou des groupes cycloalcoyle, aryle, aralcoyle ou alcaryle de 5-12 atomes de carbone.

11. Procédé selon la revendication 10, caractérisé en ce que $R^1$, $R^2$ et $R^3$ représentent des groupes alcoyle de 1-12 atomes de carbone et/ou des groupes phényle éventuellement substitués par un ou plusieurs groupes méthyle ou éthyle.

12. Procédé selon les revendications 1-11, caractérisé en ce que la réaction est conduite à des températures comprises entre 110 et 225°C, en particulier entre 125 et 200°C.